(19) Europäisches Patentamt
European Patent Office
Office européen des brevets

(11) Publication number: **0 299 726 B1**

(12) **EUROPEAN PATENT SPECIFICATION**

(45) Date of publication of patent specification: **27.10.93**

(51) Int. Cl.$^5$: **G03C 7/36**, C07D 257/04

(21) Application number: **88306373.7**

(22) Date of filing: **13.07.88**

(54) **Benzoyl-acetanilide couplers and photographic materials and processes containing them.**

(30) Priority: **17.07.87 GB 8716977**

(43) Date of publication of application:
**18.01.89 Bulletin 89/03**

(45) Publication of the grant of the patent:
**27.10.93 Bulletin 93/43**

(84) Designated Contracting States:
**DE FR GB IT NL**

(56) References cited:
**EP-A- 0 117 511**
**EP-A- 0 204 175**

**PATENT ABSTRACTS OF JAPAN, vol. 8, no. 55 (P-260)[1492], 13th March 1984**

**T.H. James, The Theory of the Photographic Process, 4th edition, 1977, pp. 354-355**

**Chemical Reviews, vol. 91, 1991, pp. 165-195**

(73) Proprietor: **EASTMAN KODAK COMPANY (a New Jersey corporation)**
**343 State Street**
**Rochester New York 14650(US)**
(84) Designated Contracting States:
**DE FR IT NL**

(73) Proprietor: **KODAK LIMITED**
**Kodak House**
**Station Road**
**Hemel Hempstead HP1 1JU**
**Hertfordshire(GB)**
(84) Designated Contracting States:
**GB**

(72) Inventor: **Southby, David Thomas**
**28 Exeter Road**
**Rayners Lane**
**Harrow Middlesex(GB)**

(74) Representative: **Baron, Paul Alexander Clifford et al**
**Kodak Limited**
**Patent Department**
**Headstone Drive**
**Harrow Middlesex HA1 4TY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services
(3.10/3.6/3.3.1)

## Description

This invention relates to benzoyl-acetanilide yellow dye-forming photographic couplers which release a photographically useful group on coupling with oxidized color developing agent and to photographic materials containing them.

It is known that desirable photographic effects can be obtained in color photography if the couplers involved in image formation release a photographically useful group on coupling with oxidized color developing agent. Examples of such couplers are those which contain the radical of a development inhibitor (DIR couplers) or a masking dye. Such couplers are of the form:

COUPLER - PUG

wherein PUG is the radical of a photographic useful group and is attached to the COUPLER at its coupling position and thus released on coupling.

US Patent 4,248,962 describes couplers of the form:

COUPLER - TIME PUG

in which, on coupling, TIME - PUG is released and, after a short delay, PUG is released from TIME (a timing group). Such couplers which release a development inhibitor have been called development inhibitor anchimeric release couplers (DIAR couplers). Thus the photographically useful group's release is delayed leading to desirable photographic effects. The specification refers generally to all classes of coupler. Coupler No. 28 (column 29) has the formula:

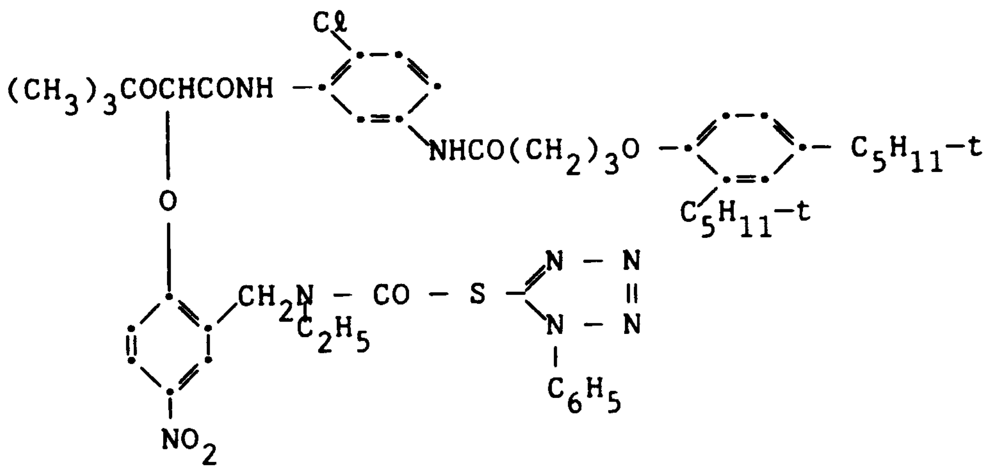

and coupler Nos. 29 and 30 are similar, all of which have a development inhibitor moiety attached via a TIME group to the coupling position.

German OLS 3,518,231A1 discloses a pivaloylacetanilide having a TIME - PUG group as described in US 4,248,962.

Japanese specification JP-A-58205150 describes ballasted alkoxybenzoylacetanilide couplers having development inhibitor moieties attached via a timing group. However, the ballast group on the anilide moiety is a -CONH-aryl group whose electron-withdrawing capability is modest. The present invention employs ballast groups which are much stronger electron-withdrawing groups and give increased activity which, in turn, provides quicker release of the time-inhibitor moiety enabling it to diffuse further during development thus having more effect on distant receiver layers.

We have now found a class of benzoyl-acetanilide couplers which are more effective than the pivaloylacetanilide DIAR couplers of the prior art.

The present invention provides a color photographic element comprising a support bearing at least one photographic silver halide emulsion layer and a non-diffusible photographic color coupler of the formula:

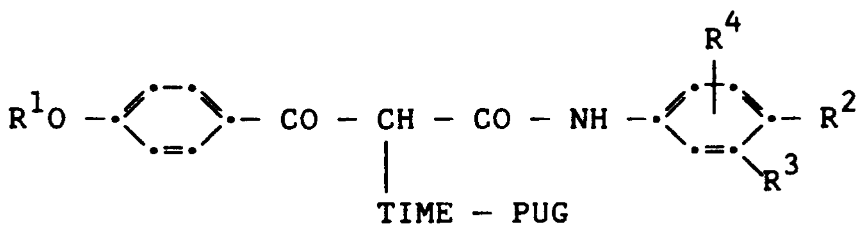

wherein TIME is a group of the formula:

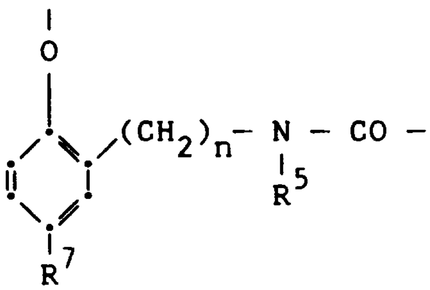

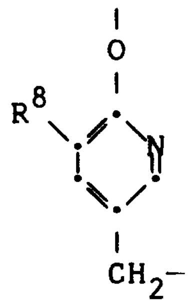

or

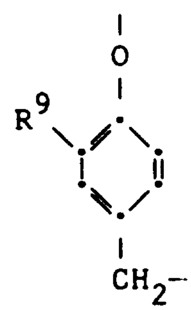

$R^1$ is an alkyl group,
$R^2$ and $R^3$ are each hydrogen or an electron-withdrawing group of the formula:

$-SO_2-NH-R^6$ $-NO_2$
$-SO-R^6$ $-CN$
$-SO_2-R^6$ $-CF_3$
$-CO-R^6$ $-OP(OR^6)_2$
$-CO_2R^6$ or $-OSO_2R^6$

provided that at least one of them is not hydrogen,

$R^4$ represents hydrogen or one or more substituents,

$R^5$ is an alkyl group of 1-4 carbon atoms or a hydroxyphenyl group,

$R^6$ is an alkyl group,

$R^7$ is an $-NO_2$, $-NHSO_2CH_3$, $-NHCOCH_3$, $-SO_2NHCH_3$, $-SO_2CH_3$ or -CN group,

$R^8$ is hydrogen, chloro or a methyl group,

$R^9$ is hydrogen or $R^7$,

PUG is the radical of a photographically useful compound, and

n is 0 or 1.

Preferably $R^6$ is an alkyl group of 1-20 carbon atoms.

Examples of $R^1$ alkyl groups are methyl, ethyl, n-butyl, t-butyl, hexyl, dodecyl and hexadecyl.

It will be understood that in order to ensure that the couplers are non-diffusible in the photographic element they will contain a group or groups of appropriate size and configuration. Such groups are usually called ballast groups and can be in groups $R^1$, $R^2$ and/or $R^3$.

The PUG group can be, for example, a radical of a development inhibitor, dye, coupler or development accelerator. Details of such groups are given in US Patents 3,227,554; 3,701,783; 3,615,506; 3,617,291; 3,379,529; 3,620,746; 3,384,657; 3,733,201; 4,248,962 and 4,409,323. Specific development inhibitor moieties include mercaptotetrazoles or, less desirably, benzotriazoles and benzodiazoles. Examples of inhibitor moieties include the following:

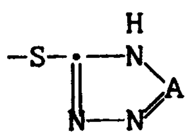

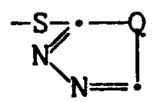

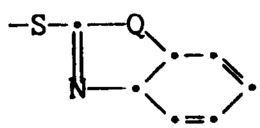

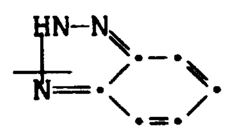

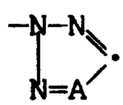

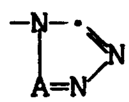

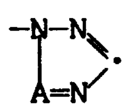

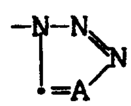

wherein

A is N or CR,

Q is O, S or NR, and

R is H or an alkyl group.

Table 1 below lists two specific examples of couplers according to the present invention together with two comparative couplers G and H.

Table 1

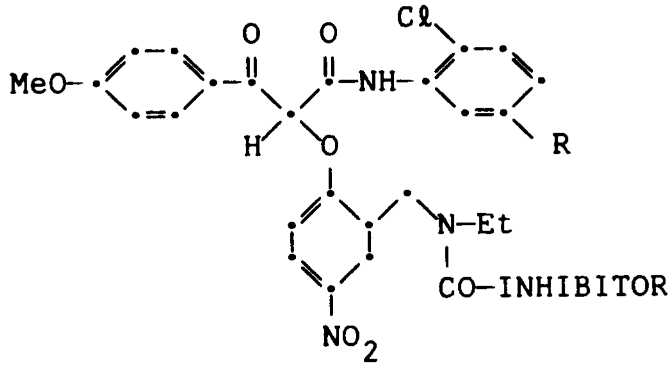

| Compound | INHIBITOR | R |
| --- | --- | --- |
| (1) | 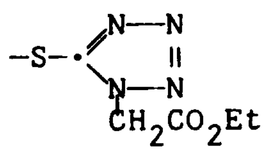 | $SO_2NHC_{16}H_{33}$ |
| (2) | 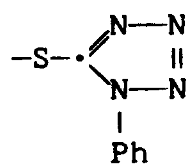 | $SO_2NHC_{16}H_{33}$ |
| (G) | 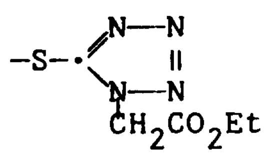 | $NHSO_2C_{16}H_{33}$ |
| (H) | 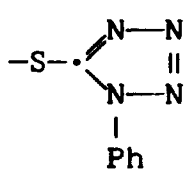 | $NHSO_2C_{16}H_{33}$ |

The dye-forming couplers of this invention can be used in the ways and for the purposes that similar dye-forming couplers have been previously used in the photographic art. They may be incorporated into photographic materials, for example, by dissolving in a suitable high and/or low boiling solvent and forming a dispersion in gelatin.

Typically, the couplers are incorporated in photographic elements associated with a silver halide emulsion. As used herein, the term "associated with" signifies that the coupler is in the silver halide emulsion layer or in an adjacent location where, during processing, it will come into reactive association with silver halide development products.

Frequently the present DIAR couplers will be associated with the yellow dye image forming layers so that any yellow image formed therefrom will form part of the yellow image. However, this is not essential and where a small amount of yellow can be tolerated, it may be associated with other layers, for example the magenta image dye forming layers.

The photographic elements can be single color elements or multicolor elements. In a multicolor element, yellow dye-forming couplers, for example, would usually be associated with a blue-sensitive emulsion, although they could be associated with an emulsion sensitized to a different region of the spectrum, or with a panchromatically sensitized, orthochromatically sensitized or unsensitized emulsion.

Multicolor elements contain dye image-forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art.

A typical multicolor photographic element would comprise a support bearing a yellow dye image-forming unit comprised of at least one blue-sensitive silver halide emulsion layer having associated therewith at least one yellow dye-forming coupler, and magenta and cyan dye image-forming units comprising at least one green- or red-sensitive silver halide emulsion layer having associated therewith at least one magenta or cyan dye-forming coupler respectively. The element can contain additional layers, such as filter layers.

In the following discussion of suitable materials for use in the emulsions and elements of this invention, reference will be made to Research Disclosure, December 1978, Item 17643, published by Industrial Opportunities Ltd., The Old Harbourmaster's, 8 North Street, Emsworth, Hants P010 7DD, U.K. This publication will be identified hereafter as "Research Disclosure".

The silver halide emulsion employed in the elements of this invention can be either negative-working or positive-working. Suitable emulsions and their preparation are described in Research Disclosure Sections I and II and the publications cited therein. Suitable vehicles for the emulsion layers and other layers of elements of this invention are described in Research Disclosure Section IX and the publications cited therein.

In addition to the couplers of this invention, the materials of the invention can include additional couplers as described in Research Disclosure Section VII, paragraphs D, E, F and G and the publications cited therein. The couplers of this invention and any additional couplers can be incorporated in the elements and emulsions as described in Research Disclosures of Section VII, paragraph C and the publications cited therein.

The photographic materials of this invention or individual layers thereof, can contain brighteners (see Research Disclosure Section V), antifoggants and stabilizers (see Research Disclosure Section VI), antistain agents and image dye stabilizer (see Research Disclosure Section VII, paragraphs I and J), light absorbing and scattering materials (see Research Disclosure Section VIII), hardeners (see Research Disclosure Section XI), plasticizers and lubricants (see Research Disclosure Section XII), antistatic agents (see Research Disclosure Section XIII), matting agents (see Research Disclosure Section XVI) and development modifiers (see Research Disclosure Section XXI).

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the element with a color developing agent to reduce developable silver halide and oxidize the color developing agent. Oxidized color developing agent in turn reacts with the coupler to yield a dye.

Preferred color developing agents are p-phenylene diamines. Especially preferred are 4-amino-3-methyl-N,N-diethylaniline hydrochloride, 4-amino-3-methyl-N-ethyl-N-$\beta$-(methanesulphonamido)-ethylaniline sulphate hydrate, 4-amino-3-methyl-N-ethyl-N-$\beta$-hydroxyethylaniline sulphate, 4-amino-3-$\beta$-(methanesulphonamido )ethyl-N,N-diethylaniline hydrochloride and 4-amino-N-ethyl-N-(2-methoxyethyl)-m-toluidine di-p-toluene sulphonate.

With negative-working silver halide emulsions this processing step leads to a negative image. To obtain a positive (or reversal) image, this step can be preceded by development with a non-chromogenic developing agent to develop exposed silver halide, but not form dye, and then uniform fogging of the element to render unexposed silver halide developable. Alternatively, a direct positive emulsion can be employed to obtain a positive image.

Development is followed by the conventional steps of bleaching, fixing, or bleach-fixing, to remove silver and silver halide, washing and drying.

The present couplers may be prepared by the following scheme:

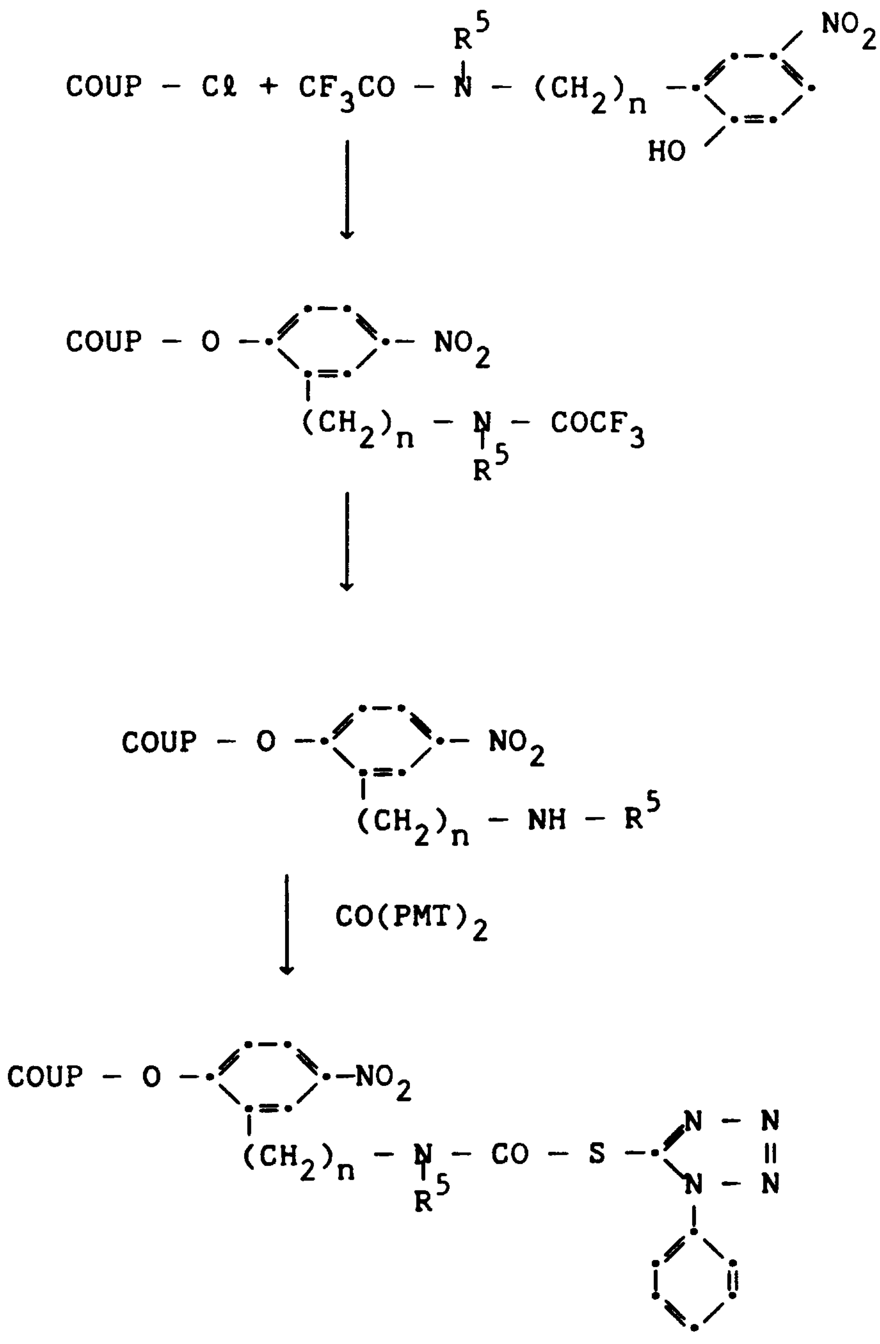


NOTE: 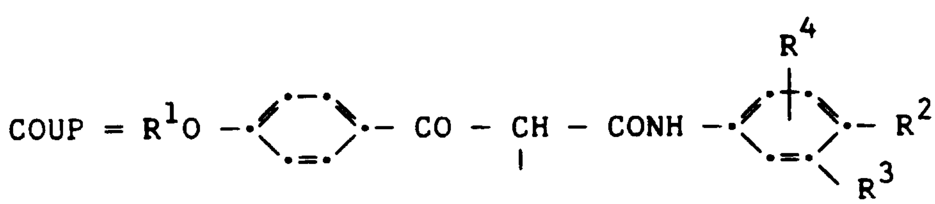

PMT = 1-phenyl-5-thiotetrazole

The first step can be carried out in dimethylformamide while the second can be carried out in alkaline dimethyl sulphoxide solution. The final step can be carried out in dry ethyl acetate using from 1 to 2

equivalents of carbonyldi-(1-phenyl-5-thiotetrazole).

The following Examples are included for a better understanding of the invention.

Example 1

Compound 2 of Table 1 above is prepared by the following route:-

(Me herein means $CH_3$-)

(Et herein means $C_2H_5$-)

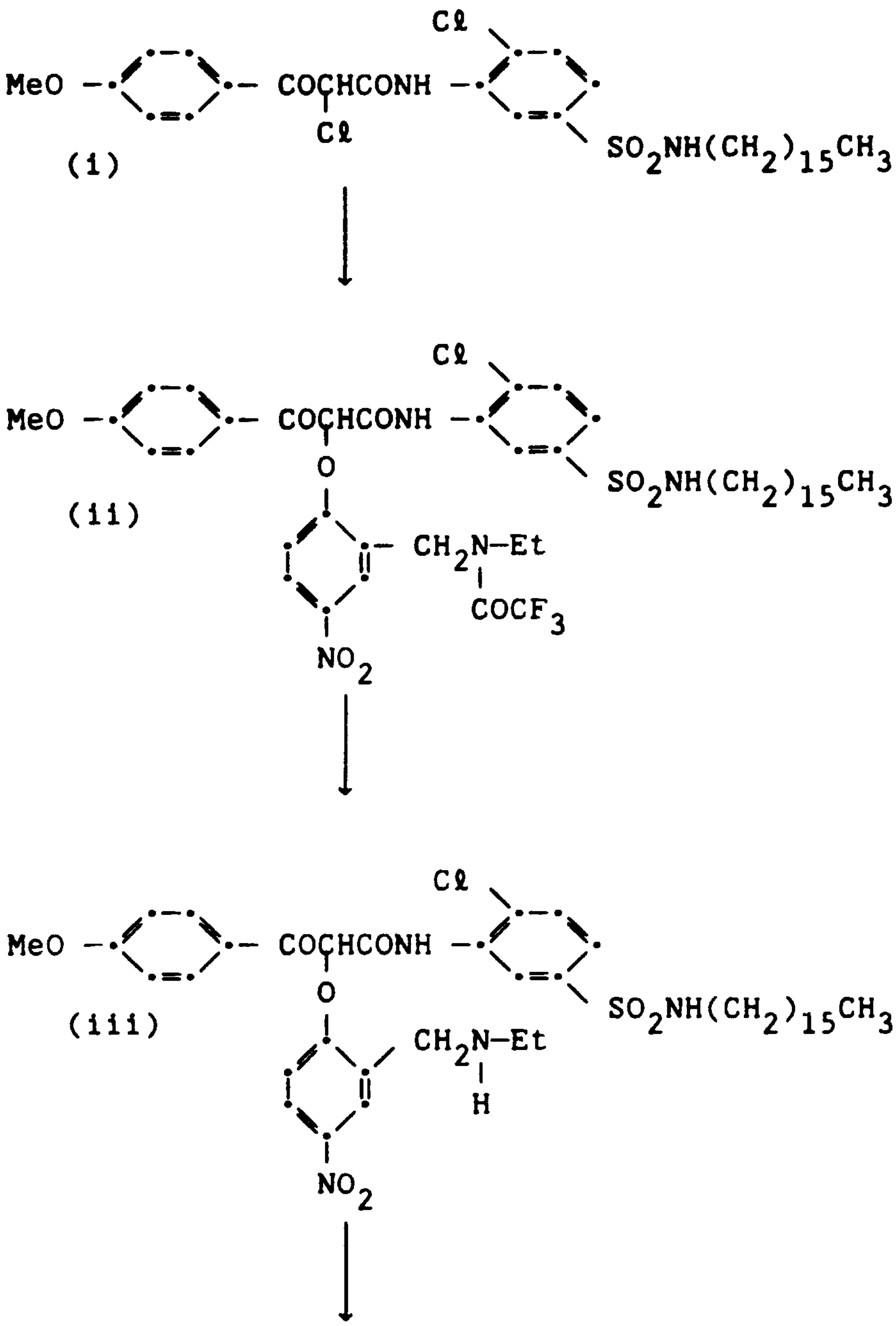

(i)

(ii)

(iii)

(2)

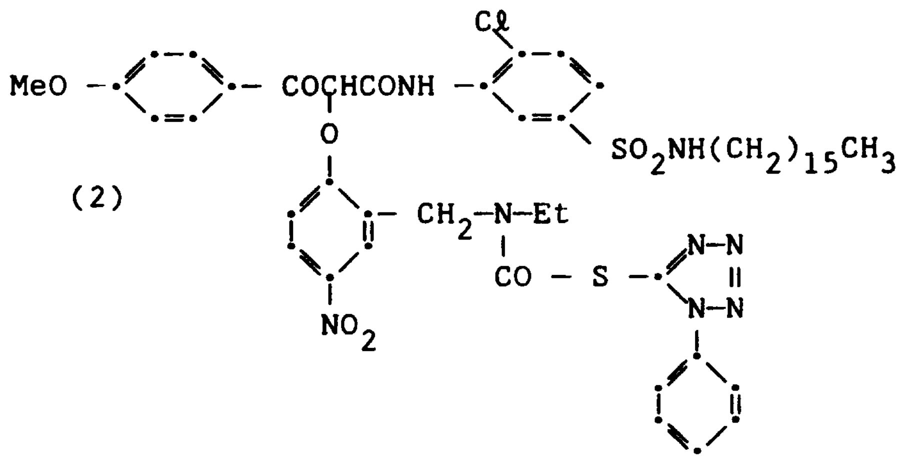

Compound (ii)

The two equivalent coupler (i) (25g, 39mmol) was taken up in dry dimethylformamide (250cm$^3$) and N-ethyl-N-(2-hydroxy-5-nitrophenylmethyl)-trifluoro acetamide (46.5g, 125mmol) was added. The solution was flushed with nitrogen for thirty minutes then triethylamine (16.5cm$^3$, 117mmol) was added and the mixture was heated at 50° for 1.5 hours. Thin layer chromatography (tlc) analysis then showed that all of the starting coupler had reacted.

The reaction mixture was poured into water (3ℓ) plus conc. hydrochloric acid (150cm$^3$) and the whole was extracted with ethyl acetate (3 x 500cm$^3$). This organic extract was washed successively with 500cm$^3$ portions of brine, ice cold 1M sodium hydroxide solution (3x), brine and 3M hydrochloric acid (3x). The organic phase was dried over magnesium sulphate and concentrated to leave a glassy solid which was recrystallized from cyclohexane (330cm$^3$) to yield the product as a brittle solid 30g, 86%.

Compound (iii)

Sodium hydroxide solution (3M, 12cm$^3$) was added to dimethylsulphoxide (12cm$^3$) and the resulting solution was cooled to room temperature before it was added to a solution of (ii) (6g, 6.7mmol) in dimethylsulphoxide (120cm$^3$), stirred at room temperature under a nitrogen atmosphere. After stirring for ten minutes, tlc analysis showed that all the starting material had reacted and so the reaction mixture was quenched in dilute hydrochloric acid (water (51) plus 12.5M acid (50cm$^3$). The solid which formed was filtered off and dried to give 5.2g, 97% of crude product, which was recrystallized from a mixture of ethyl acetate (20cm$^3$) and cyclohexane (100cm$^3$) to give (iii) as a yellow solid 2.7g, 50%.

Compound 2

Compound (iii) (2.6g, 3.2mmol) was suspended in dry ethyl acetate (25cm$^3$) and solid carbonyldi(1-phenyl-5-thiotetrazole) (1.86g, 4.9mmol) was added in portions, over five minutes. A clear solution formed and it was stirred at room temperature for one hour. The solution was diluted with ethyl acetate (75cm$^3$) and the whole was washed successively with portions (100cm$^3$) of 3M sodium carbonate solution (3x), water and 3M hydrochloric acid (3x). It was then dried over magnesium sulphate and concentrated to leave a glass (2.7g).

This was taken up in pyridine (50cm$^3$) and water (25cm$^3$) was added. This mixture was heated at reflux for ten minutes, allowed to cool slightly and poured into brine (2 litres). The solid product which precipitated was collected by filtration and dried to give 2.4g, 74% of compound 2. Purification by column chromatography using toluene-ethyl acetate mixtures (1-9 to 1-4) as eluant, followed by recrystallization from ethyl acetate/petroleum ether (60-80°), yielded the product (2) as white solid with mp = 64°, hplc (detected at 275nm) = 99%,

| $C_{49}H_{61}ClN_8O_9S_2$ | | | | | |
|---|---|---|---|---|---|
| requires:<br>found: | C 58.5,<br>C 58.7, | H 6.1,<br>H 6.2, | Cl 3.5,<br>Cl 3.7, | N 11.2,<br>N 10.9, | S 6.4%;<br>S 6.1%. |

Positive Ion FAB Mass Spectrometry showed (MH)+ at 1005m/z. 1H NMR spectrum in $CDCl_3$ was consistent with the desired structure and showed about 30% enolization.

Comparative compound H was prepared, as described above, as a solid mp = 65°. Hplc (detected at 275nm) = 93% with 7% impurity or enolized coupler.

| $C_{49}H_{61}ClN_8O_9S_2$ | | | | | |
|---|---|---|---|---|---|
| requires:<br>found: | C 58.5,<br>C 58.5, | H 6.1,<br>H 6.2, | Cl 3.5,<br>Cl 3.5, | N11.2,<br>N 10.9, | S 6.4%;<br>S 6.6%. |

Positive Ion FAB Mass Sectrometry showed (MH)+ at 1005m/z. 1H NMR spectrum in $CDCl_3$ was consistent with the desired structure.

## Example 2

The performance of coupler (2) of Example 1 was compared to that of the couplers A and H. Coupler A has the formula:

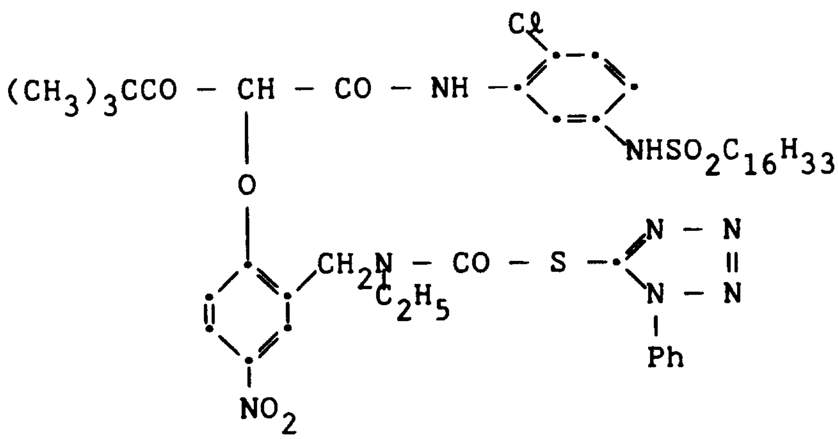

Each DIAR coupler was coated in a 2-layer, causer-receiver format coated on a photographic film base on top of a layer of gelatin containing grey colloidal silver as antihalation layer and with a protective overlayer of 5.4 g/m$^2$ of gelatin as follows (coating weights in g/m$^2$, coating weights for emulsions quoted as g/m$^2$ silver):

| | | |
|---|---|---|
| Gelatin<br>Yellow Coupler B<br>DIAR coupler<br>Green sensitized silver bromoiodide emulsion | 2.7<br>1.2<br>(varies)<br>1.6 | (Causer layer) |
| Gelatin<br>2,5-bis(1-methylundecyl)-1,4-benzenediol | 0.9<br>0.1 | |
| Gelatin<br>Coupler C<br>Red sensitized silver bromoiodide emulsion | 2.4<br>0.7<br>1.6 | (Receiver layer) |
| Support | | |

The couplers employed were as follows:

Coupler B

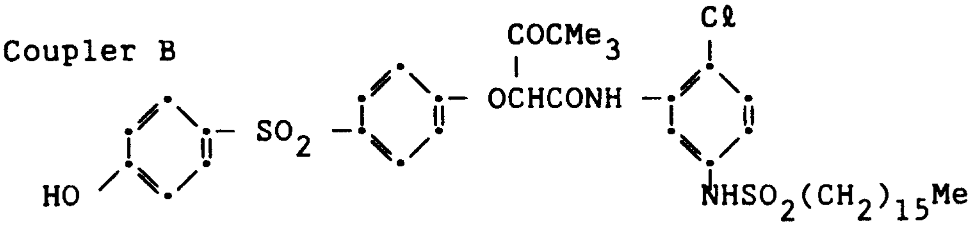

Coupler C

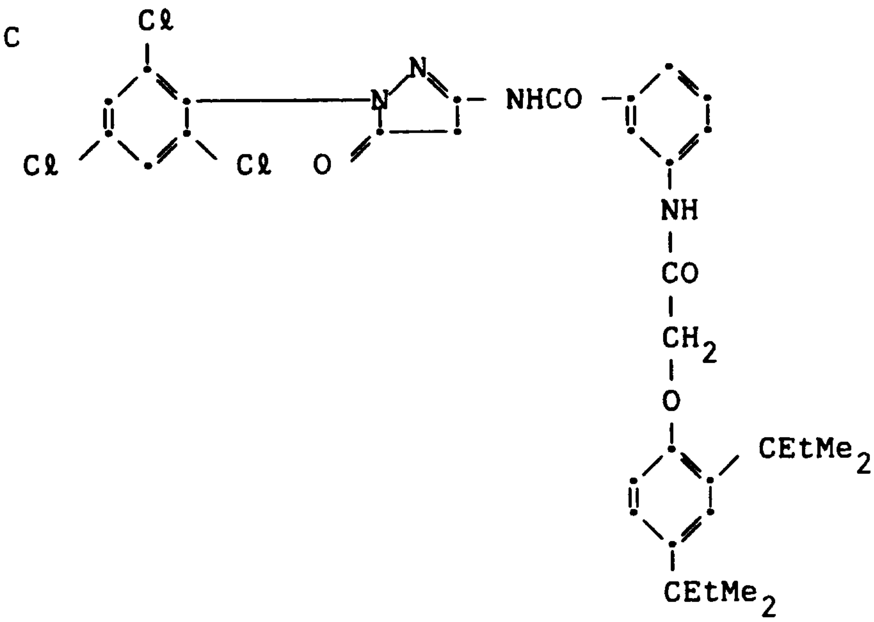

The DIAR couplers were coated at several laydowns and their effectiveness was compared to pivaloylacetanilide control couplers.

The coatings were exposed on an EASTMAN 1B sensitomer through a 1cm wide 31 step (0.4 log E) neutral density step wedge through a WRATTEN 99 filter to expose the causer layer and in an overlaid 0.5 cm wide step wedge through a WRATTEN 12 filter to expose the causer and receiver layers.

The exposed coatings were processed in the C41 processing baths described in the British Journal of Photography Annual 1977, pages 204-5 at 38 °C with a development time of 3.25 minutes. The contrast of the causer and receiver layer images was measured and the percentage contrast reduction calculated by using the formula:

$$\frac{Go - Gx}{Go} \times 100$$

wherein Go is the contrast without any DIAR coupler and Gx is the contrast in the presence of the DIAR coupler in the amount indicated in Table 2 below.

Table 2

| Coupler No moles/m²x10⁵ | | Causer Contrast | Receiver Contrast | % Causer Reduction | % Receiver Reduction |
|---|---|---|---|---|---|
| | 0 | 2.66 | 1.67 | | |
| 2 | ( 5.6 | 2.16 | 1.38 | 19 | 17 |
| | (11.3 | 1.28 | 0.94 | 52 | 44 |
| | (16.9 | 0.79 | 0.62 | 70 | 63 |
| H | ( 5.6 | 2.40 | 1.47 | 10 | 12 |
| | (11.3 | 1.56 | 1.15 | 41 | 31 |
| | (16.9 | 1.04 | 0.83 | 61 | 50 |
| A (Prior art) | ( 5.6 | 2.48 | 1.55 | 7 | 7 |
| | (11.3 | 1.85 | 1.29 | 30 | 23 |
| | (16.9 | 1.32 | 1.03 | 50 | 38 |

It will be seen that at each of the three laydowns coupler 2 of the invention shows superior contrast reduction percentages to those achieved by the comparative Couplers A and H.

Sharpness measurements were made on coatings which had matched causer contrasts. They were given a neutral exposure through a Wratten 12 filter, to expose both causer and receiver. Sharpness was evaluated by calculating CMT acutance values and AMT acutance values. The use of CMT is described by R G Gendron in "An Improved Objective Method for Rating Picture Sharpness: CMT Acutance" Journal of the SMPTE,82,1009-12, 1973 in which CMT is defined as:

$$CMT = 100 + B \log \left( \frac{\text{area of cascaded MTF}}{\text{area of eye MTF}} \right)$$

wherein B = 42.

It is believed that AMT represents more accurately the perceived sharpness in prints and is used as a measure of sharpness in the 35 mm format. AMT is defined by the same formula as CMT except that B = 66.

Thus, coatings containing 11.3 x $10^{-5}$ mole/$m^2$ of coupler 2 with causer contrast = 1.28 and 16.9 x $10^{-5}$ mole/$m^2$ of coupler A with causer contrast 1.32 were used for sharpness measurements.

Results:

| Coupler | 16mm CMT | 35mm system AMT |
|---|---|---|
| 2 | 92.1 | 93.2 |
| A | 91.1 | 92.9 |

A change in CMT or AMT of greater than one unit is considered to be indicative of a perceivable change in sharpness. On this basis Coupler A and Coupler 2 have equivalent performance when, Coupler 2 is coated at only $^2/3$ the laydown of Coupler A. Thus Coupler 2 has a reactivity advantage over the prior art Coupler A.

Example 3

Couplet 2 in a magenta dye forming layer.

A photographic material was prepared as described in Example 2 except for the following:

Causer - couplet B replaced with magenta couplet D (see below) at 0.77 g/$m^2$

Receiver - couplet C replaced by yellow couplet E (see below) at 1.3 g/m². The control coupler was the DIR couplet F (see below).

Comparison of the effects of inhibitor released from a DIR and DIAR is difficult because the timing switch leads to longer range effects. However these longer range effects do lead to stronger interimage effects and sharpness boosts especially in the larger formats. For these reasons DIARs are favored over DIRs for many applications.

Contrast Data

| DI(A)R | Gc | Gr | %Gc red | %Gr red | %r red of total |
|---|---|---|---|---|---|
| none | 2.31 | 1.24 | – | – | – |
| Coupler F | | | | | |
| $2.7 \times 10^{-5}$ | 1.40 | 1.28 | 39 | –3 | 0 |
| 5.4 | 1.02 | 1.12 | 56 | 10 | 15 |
| 10.7 | 0.61 | 0.80 | 74 | 35 | 32 |
| 21.6 | 0.41 | 0.45 | 82 | 64 | 44 |
| Coupler 2 | | | | | |
| $2.7 \times 10^{-5}$ | 1.81 | 1.35 | 22 | –9 | 0 |
| 5.3 | 1.33 | 1.02 | 42 | 18 | 30 |
| 10.7 | 0.58 | 0.39 | 75 | 69 | 48 |
| 21.4 | 0.19 | 0.24 | 92 | 81 | 47 |

NB. where contrast increases have occurred, negative values of %Gr red receiver are given.

The % receiver reduction of the total % reduction is given by:-

$$\frac{\%Gr}{\%Gc + \%Gr}$$

and is given as zero when negative values occur.

At the $10.7 \times 10^{-5}$ moles/m² level of Coupler F and Coupler 2, the causer contrasts are closely matched (0.61 versus 0.58). Here Coupler 2 shows more receiver contrast reduction 48% to 32% for Coupler F. So Coupler 2 and Coupler F may be considered to have approximately the same activity in the causer but Coupler 2 holds an advantage in the receiver.

Sharpness data on these two coatings are as follows:-

| DI(A)R | 16mm CMT | 35mm system AMT |
|---|---|---|
| F | 95.5 | 94.4 |
| 2 | 96.8 | 96.7 |
| gain | 1.3 | 2.3 |

These show that coupler 2 shows a perceivable improvement in sharpness in 16mm format but shows a more distinct advantage in the larger 35mm format, where the timed release of the inhibitor from the DIAR becomes more important.

STRUCTURES

Coupler D

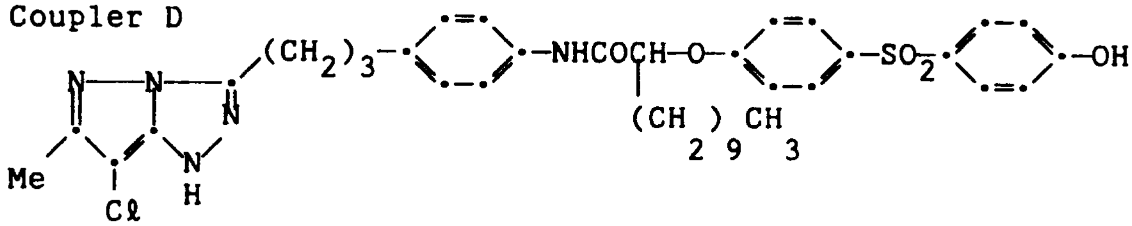

Coupler E

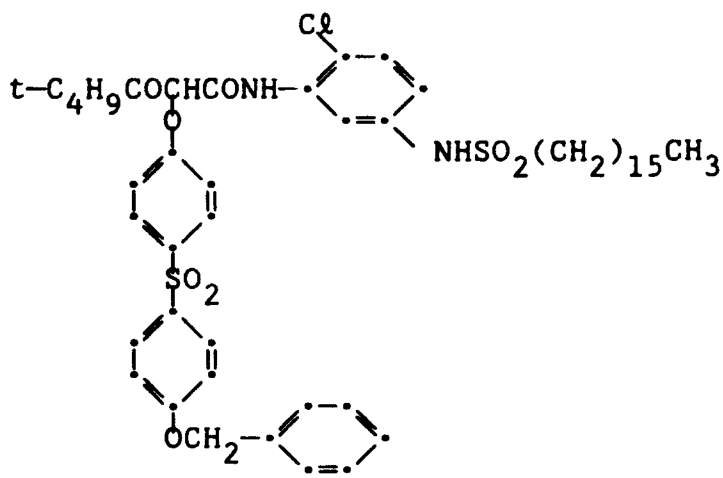

Coupler F

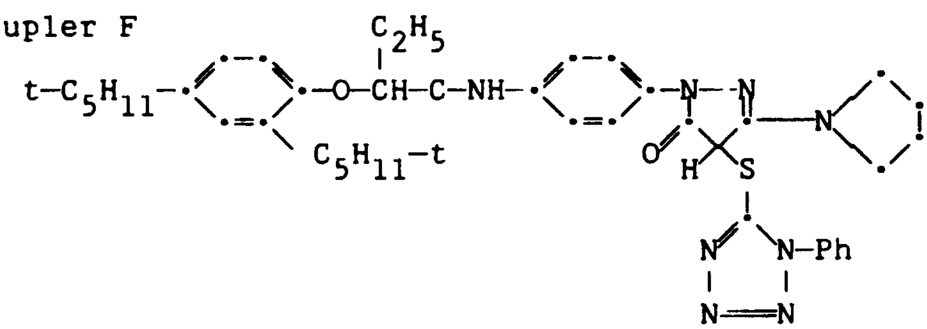

## Claims

**1.** A non-diffusible photographic colour coupler of the general formula:

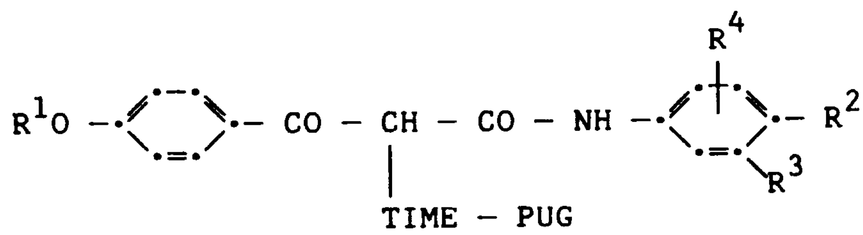

in which TIME is a group of the formula:

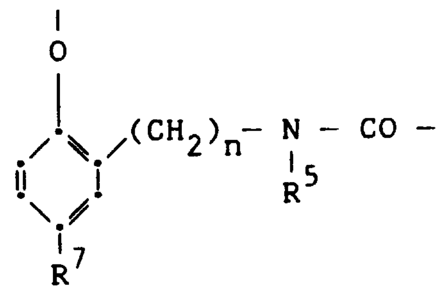

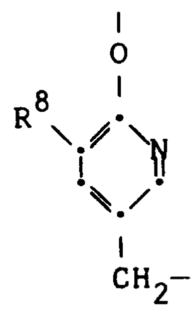

or

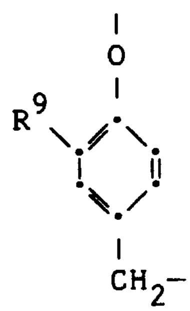

$R^1$ is an alkyl group,
$R^2$ and $R^3$ are each hydrogen or an electron-withdrawing group of the formula:

$-SO_2-NH-R^6$ $-NO_2$
$-SO-R^6$ $-CN$

$-SO_2-R^6$ $-CF_3$
$-CO-R^6$ $-OP(OR^6)_2$
$-CO_2R^6$ or $-OSO_2R^6$

provided that at least one of them is not hydrogen,
$R^4$ represents hydrogen or one or more substituents,
$R^5$ is an alkyl group of 1-4 carbon atoms or a hydroxyphenyl group,
$R^6$ is an alkyl group,
$R^7$ is an $-NO_2$, $-NHSO_2CH_3$, $-NHCOCH_3$, $-SO_2NHCH_3$, $-SO_2CH_3$ or -CN group,
$R^8$ is hydrogen, chloro or a methyl group,
$R^9$ is hydrogen or $R^7$,
PUG is the radical of a photographically useful compound, and
n is 0 or 1.

**2.** A coupler as claimed in Claim 1 in which $R^6$ is an alkyl group of 1-20 carbon atoms.

**3.** A coupler as claimed in Claim 1 or 2 in which PUG represents the radical of a development restrainer or a development accelerator.

**4.** A coupler as claimed in Claim 3 in which PUG is the radical of 5-mercapto tetrazole.

**5.** A coupler according to Claim 1 wherein the coupler is represented by the formula:

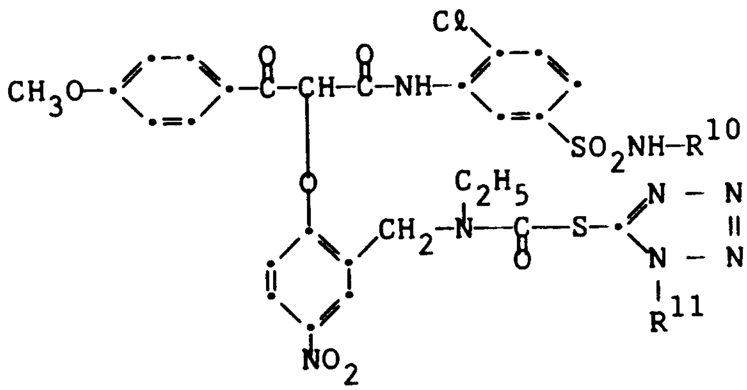

wherein $R^{10}$ is an alkyl group of 1-20 c atoms and, $R^{11}$ is an alkyl or aryl group.

**6.** A photographic coupler represented by the formula:

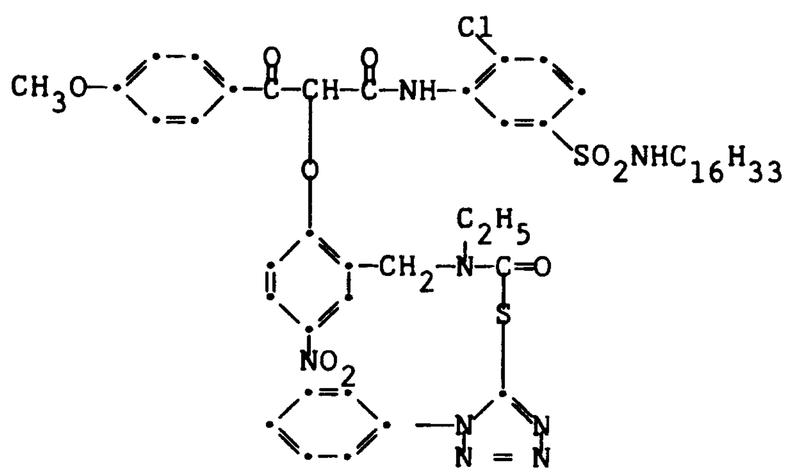

7. A photographic photosensitive material comprising a support bearing at least one silver halide emulsion layer having associated therewith a coupler according to any of Claims 1 - 6.

8. A multicolour photographic photosensitive material as claimed in Claim 7 comprising a support bearing a yellow dye image-forming unit comprised of at least one blue-sensitive silver halide emulsion layer having associated therewith at least one yellow dye-forming coupler, and magenta and cyan dye image-forming units comprising at least one green- or red-sensitive silver halide emulsion layer having associated therewith at least one magenta or cyan dye-forming coupler respectively wherein at least one emulsion layer thereof has associated therewith a coupler according to any of Claims 1 - 6.

**Patentansprüche**

1. Nicht-diffundierender photographischer Farbkuppler der allgemeinen Formel:

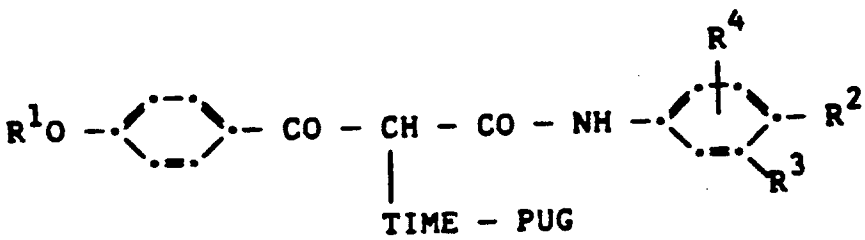

worin TIME steht für eine Gruppe der Formeln:

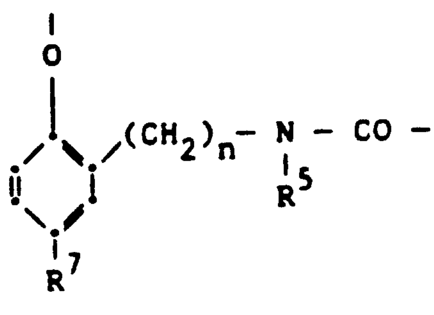

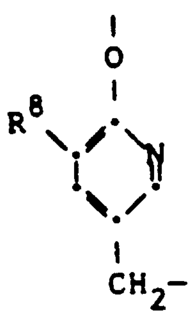

oder

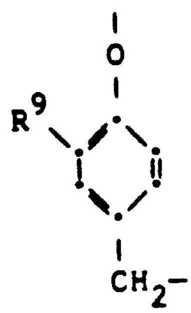

und worin ferner bedeuten: $R^1$ eine Alkylgruppe, $R^2$ und $R^3$ jeweils Wasserstoff oder eine Elektronen abziehende Gruppe der Formeln:

$-SO_2-NH-R^6$ $-NO_2$
$-SO-R^6$ $-CN$
$-SO_2-R^6$ $-CF_3$
$-CO-R^6$ $-OP(OR^6)_2$
$-CO_2R^6$ oder $-OSO_2R^6$

wobei gilt, daß mindestens einer der Reste nicht aus Wasserstoff besteht,
$R^4$ Wasserstoff oder einen oder mehrere Substituenten,
$R^5$ eine Alkylgruppe mit 1 bis 4 Kohlenstoffatomen oder eine Hydroxyphenylgruppe,
$R^6$ eine Alkylgruppe,
$R^7$ eine der folgenden Gruppen $-NO_2$, $-NHSO_2CH_3$, $-NHCOCH_3$, $-SO_2NHCH_3$, $-SO_2CH_3$ oder -CN,
$R^8$ Wasserstoff, Chlor oder eine Methylgruppe,
$R^9$ Wasserstoff oder $R^7$,
PUG einen Rest einer photographisch verwendbaren Verbindung und
n gleich 0 oder 1.

2. Kuppler nach Anspruch 1, in dem $R^6$ für eine Alkylgruppe mit 1 bis 20 Kohlenstoffatomen steht.

3. Kuppler nach Anspruch 1 oder 2, in dem PUG für den Rest eines die Entwicklung hemmenden Mittels oder den Rest eines Entwicklungsbeschleunigers steht.

4. Kuppler nach Anspruch 3, in dem PUG für den Rest von 5-Mercaptotetrazol steht.

5. Kuppler nach Anspruch 1 mit der folgenden Formel:

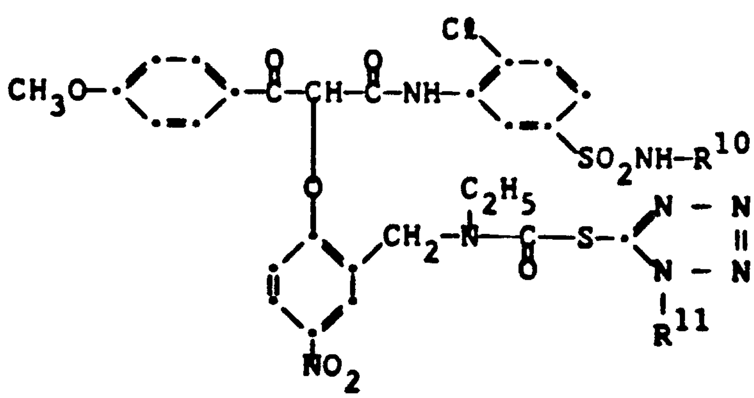

worin $R^{10}$ für eine Alkylgruppe mit 1 bis 20 C-Atomen steht und $R^{11}$ für eine Alkyl- oder Arylgruppe.

**6.** Photographischer Kuppler mit der folgenden Formel:

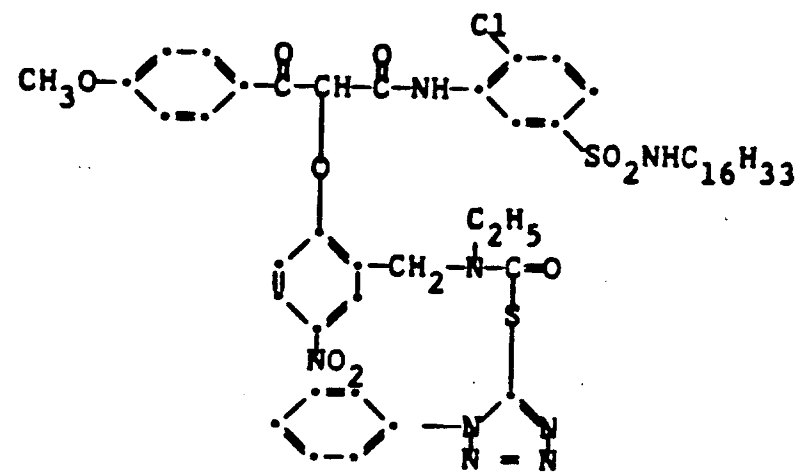

**7.** Photographisches photosensitives Material mit einem Träger, auf dem mindestens eine Silberhalogenidemulsionsschicht aufgetragen ist, der ein Kuppler nach einem der Ansprüche 1 bis 6 zugeordnet ist.

**8.** Mehrfarbiges photographisches photosensitives Material nach Anspruch 7 mit einem Träger, auf den aufgetragen ist eine ein gelbes Farbstoffbild liefernde Einheit mit mindestens einer blau-empfindlichen Silberhalogenidemulsionsschicht, der mindestens ein einen gelben Farbstoff liefernder Kuppler zugeordnet ist, sowie purpurrote und blaugrüne Farbstoffbilder liefernde Einheiten mit mindestens einer grün- oder rot-empfindlichen Silberhalogenidemulsionsschicht, der mindestens ein einen purpurroten oder einen blaugrünen Farbstoff bildender Kuppler zugeordnet ist, wobei mindestens einer Emulsionsschicht ein Kuppler nach einem der Ansprüche 1 bis 6 zugeordnet ist.

**Revendications**

**1.** Un coupleur photographique coloré ne diffusant pas, de formule générale :

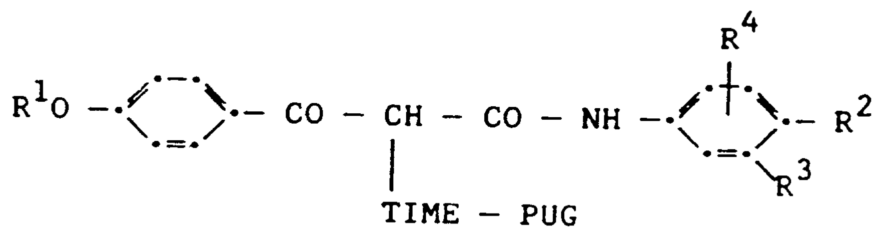

dans laquelle TIME est un groupe de formule :

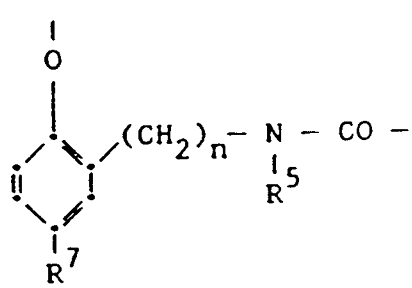

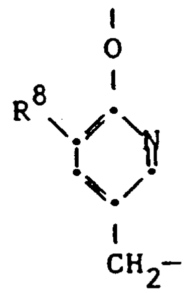

ou

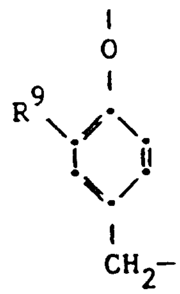

$R^1$ est un groupe alkyle,
$R^2$ et $R^3$ sont chacun un hydrogène ou un groupe attracteur d'électron de formule :

$-SO_2-NH-R^6$ $-NO_2$
$-SO-R^6$ $-CN$
$-SO_2-R^6$ $-CF_3$
$-CO-R^6$ $-OP(OR^6)_2$
$-CO_2R^6$ ou $-OSO_2R^6$

à la condition supplémentaire qu'au moins l'un d'entre eux ne soit pas un hydrogène,
$R^4$ est un hydrogène ou un ou plusieurs substituants,
$R^5$ est un groupe alkyle avec 1 à 4 atomes de carbone ou un groupe hydroxyphényle,
$R^6$ est un groupe alkyle,
$R^7$ est un groupe $-NO_2$, $NHSO_2CH_3$, $-NHCOCH_3$, $-SO_2NHCH_3$, $-SO_2CH_3$ ou -CN,
$R^8$ est un hydrogène, un chlore ou un groupe méthyle,
$R^9$ est un hydrogène ou un $R^7$,
PUG est le radical d'un composé photographiquement utile, et n est 0 ou 1.

**2.** Un coupleur selon la revendication 1 dans laquelle $R^6$ est un groupe alkyle avec 1 à 20 atomes de carbone.

**3.** Un coupleur selon la revendication 1 ou 2 dans laquelle PUG est le radical d'un retardateur ou d'un accélérateur de développement.

**4.** Un coupleur selon la revendication 3 dans laquelle PUG est le radical 5-mercapto tétrazole.

**5.** Un coupleur selon la revendication 1 dont le coupleur est représenté par la formule :

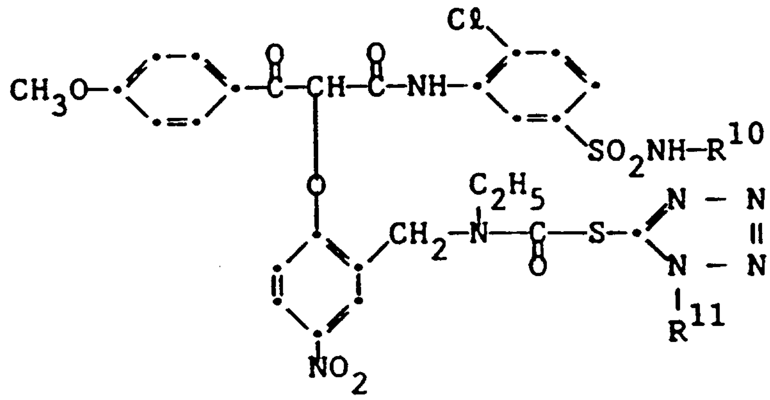

ou $R^{10}$ est un groupe alkyle avec 1 à 20 atomes de carbone et $R^{11}$ est un groupe alkyle ou aryle.

**6.** Un coupleur photographique représenté par la formule :

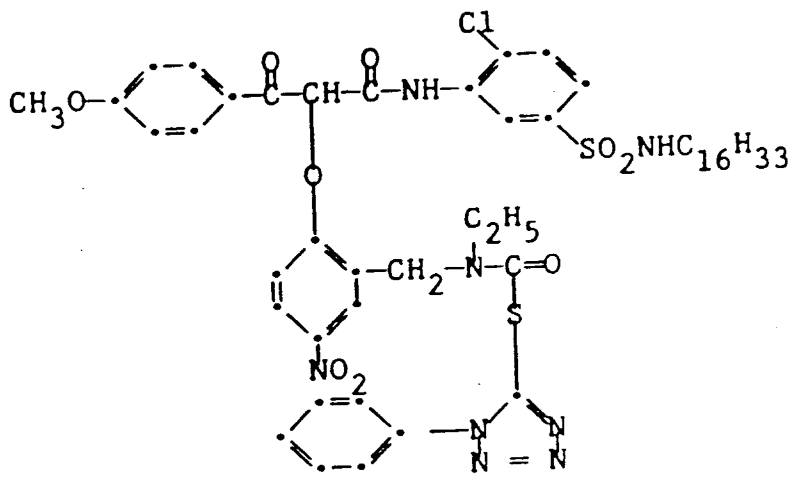

**7.** Un produit photographique photosensible comprenant un support recouvert d'au moins une couche d'émulsion d'halogénure d'argent associée avec un coupleur selon l'une des revendications 1 à 6.

**8.** Un produit photographique photosensible multicolore selon la revendication 7 comprenant un support portant une unité formatrice d'image de colorant jaune avec au moins une couche d'émulsion aux halogénures d'argent sensible au bleu associée avec au moins un coupleur formant un colorant jaune, et des unités formatrices d'images de colorants magenta et cyan comprenant au moins une couche d'émulsion aux halogénures d'argent sensible au vert ou au rouge associée avec au moins un coupleur formant un colorant respectivement magenta ou cyan, dans lequel au moins une couche d'émulsion est associée avec un coupleur selon l'une des revendications 1 à 6.